# EUROPEAN PATENT SPECIFICATION

(11) **EP 3 292 112 B1**
(45) Date of publication and mention of the grant of the patent: **14.08.2019**
(21) Application number: 16738885.9
(22) Date of filing: 03.05.2016
(51) Int. Cl.: C07D 401/04, C07C 51/41, C07C 63/08

(54) **PROCESS FOR THE PREPARATION OF ALOGLIPTIN**
VERFAHREN ZUR HERSTELLUNG VON ALOGLIPTIN
PROCÉDÉ POUR LA PRÉPARATION D' ALOGLIPTINE

(30) Priority: 04.05.2015 IN 1772MU2015
(43) Date of publication of application: 14.03.2018
(73) Proprietor: Indoco Remedies Limited, Mumbai 400 098 MAH (IN)
(72) Inventor: NAIR, Ranjeet, Navi Mumbai 400701 (IN); RAMESAN, Palangat Vayalileveetil, Navi Mumbai 400701 (IN); PANANDIKAR, Aditi Milind, Mumbai 400098 (IN)
(74) Representative: Rees, Kerry
(86) International application number: PCT/IN2016/050122
(87) International publication number: WO 2016/178246

(56) References cited:
- EP-A1- 2 682 110
- WO-A1-2010/109468
- WO-A2-2007/035629
- CN-A- 104 151 253
- CN-A- 104 447 685
- WANG, Y. ET AL.: "Alogliptin benzoate", DRUGS OF THE FUTURE, vol. 33, no. 1, 2008, pages 7-12, XP55297822, ISSN: 0377-8282, DOI: 10.1358/dof.2008.033.01.1171514

## Description

### FIELD OF INVENTION:

The present invention relates to a novel process for the preparation of alogliptin and its pharmaceutically acceptable salt.

### BACKGROUND OF THE INVENTION:

The compound, 2-[[6-[(3R)-3-amino-1-piperidinyl]-3,4-dihydro-3-methyl-2,4-dioxo-1(2H)-pyrimidinyl]methyl]benzonitrile commonly known as alogliptin represented below as the compound of Formula I, is a dipeptidyl peptidase-4 inhibitor (DPP-4) that is designed to slow the inactivation of incretin hormones glucagon-like peptide-1 (GLP-1) and glucose-dependent insulinotropic peptide (GIP).

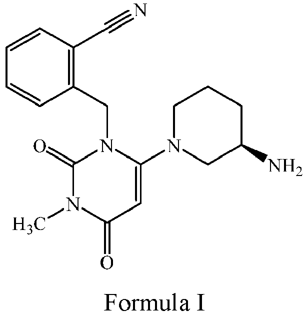

DPP-4 inhibitors are a class of oral hypoglycemic that block DPP-4 and are very effective and useful for treating Type 2 diabetes mellitus. This class of antidiabetic drugs offers some advantages over existing oral options for the management of type 2 diabetes such as a negligible risk of hypoglycemia compared with sulfonylureas and, in general, a weight-neutral profile. DPP-4 inhibitors such as sitagliptin, vildagliptin, saxagliptin, linagliptin, alogliptin and teneligliptin are currently in clinical use. These DPP-4 inhibitors are commonly known as gliptins. Like other gliptins, alogliptin causes little or no weight gain and exhibits relatively modest glucose-lowering activity. Alogliptin benzoate is sold by Takeda Pharma under the trade name Nesina®in the US, and Vipidia® in Europe.

Alogliptin and its pharmaceutical acceptable salts and process for their preparation are described in the patents, US 7,807,689 ("US'689 Patent) and its corresponding European patent, EP 1586571. The process described in US'689 Patent involves reacting 6-chlorouracil with α-bromo-o-tolunitrile in the presence of strong alkali such as sodium hydride (NaH) and lithium bromide (LiBr) in a mixture of solvents such as dimethylformamide (DMF) and dimethylsulfoxide (DMSO) to yield the intermediate compound, 2-(6-chloro-2,4-dioxo-3,4-dihydro-2H-pyrimidin-1-ylmethyl)benzonitrile. The intermediate compound on methylation using methyl iodide (MeI) in the presence of sodium hydride and a mixture of solvents, dimethylformamide (DMF) and tetrahydrofuran (THF) yields N-methylated intermediate compound, 2-[(6-chloro-3-methyl-2,4-dioxo-3,4-dihydro-2H-pyrimidin-1-yl)methyl]benzonitrile. The N-methylated intermediate compound on reaction with (R)-3-amino-piperidine dihydrochloride in the presence of sodium bicarbonate and a solvent such as methanol yields alogliptin free base, the compound of Formula I. The above process for the preparation of alogliptin is schematically represented in the following Scheme I.

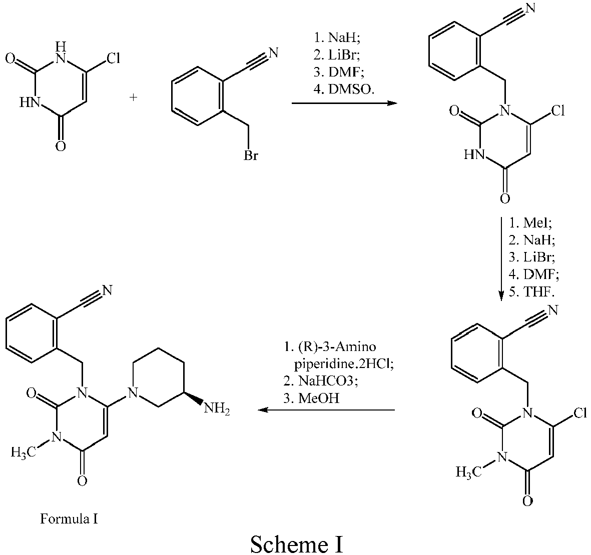

Another patent, US 8,222,411 describes a process for the preparation of alogliptin and its pharmaceutically acceptable salts wherein the compound 6-chloro-3-methyluracil is reacted with α-bromo-o-tolunitrile to give the intermediate compound, 2-[(6-chloro-3-methyl-2,4-dioxo-3,4-dihydro-2H-pyrimidin-1-yl)methyl]benzonitrile. The intermediate compound is then reacted with (R)-3-aminopiperidine dihydrochloride in isopropanol and water in the presence of potassium carbonate to get alogliptin hydrochloride, which on reaction with potassium carbonate followed by reaction with benzoic acid yields the benzoate salt of alogliptin, the compound of Formula I.

PCT Patent Application No. WO2010109468 describes preparation of alogliptin and its pharmaceutically acceptable salt wherein the urea derivative of compound A is reacted with malonic acid derivative or cyanoacetic acid derivative to give compound B or B1. The compound B or B1 on reaction with halogenating agent yields the compound C, 6-halopyrimidinedione, which on reaction with Boc protected (R)-3-aminopiperidine, yields protected alogliptin free base of compound D. The compound D on reaction with an acid results in corresponding acid addition salt of alogliptin. The reaction sequence is as shown in Scheme II below. 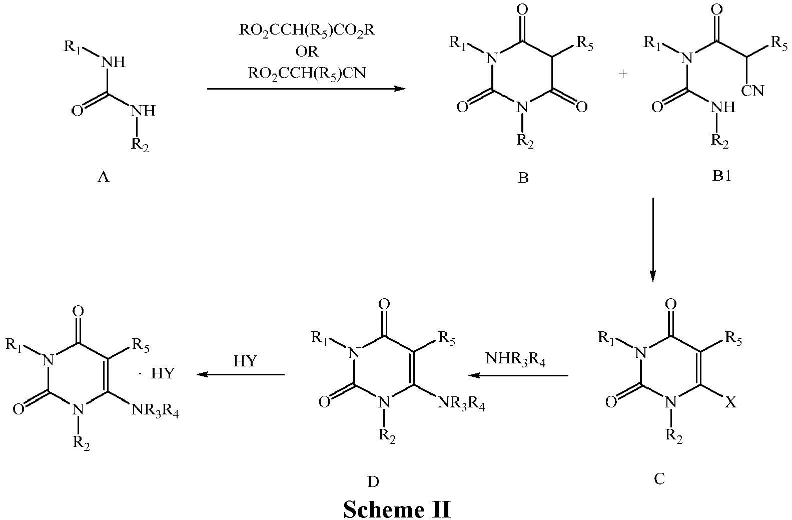 wherein: R₁ and R₅ are each independently H or (C₁-C₁₀) alkyl; R₂ is CH₂Ar; and R₃ and R₄ together with the nitrogen to which they are attached form a 4, 5, 6 or 7 membered ring, which may be unsubstituted or substituted.

Another published patent application WO2013046229 describes the preparation of amorphous alogliptin benzoate and novel acid addition salts of alogliptin i.e., fumarate, maleate, sulfate, tosylate, oxalate, nitrate, crystalline hydrochloride & crystalline tartrate.

CN 104 151 253 discloses a method for synthesizing Alogliptin intermediate 2-[(6-chlorine-3,4-dihydro-3-methyl-2,4-dioxo-1(2H)-pyrimidyl) methyl] cyanobenzene, and belongs to the field of preparation methods of chemical drug intermediates. The preparation method comprises the steps: (1) obtaining 1-methyl barbituric acid through reaction of methylurea with malonic acid; (2) obtaining 3-methyl-6-chlorouracil through chlorination reaction of 1-methyl barbituric acid and phosphorus oxychloride; and (3) obtaining Alogliptin intermediate 2-[(6-chlorine-3,4-dihydro-3-methyl-2,4-dioxo-1(2H)-pyrimidyl) methyl] cyanobenzene through reaction of 3-methyl-6-chlorouracil with 2-cyanobenzyl bromide.

CN 104 447 685 discloses a preparation method of alogliptin. The preparation method comprises the steps of: performing cyclization of N-methylurea with malonic acid derivative to obtain compound shown in the formula III, introducing a leaving group to obtain a compound shown in the formula IV, reacting with a compound shown in the formula V to obtain a compound shown in the formula VI, then reacting the compound shown in the formula VI and (R)-3-Boc aminopiperidine to obtain Boc-protectec alogliptin the compound shown in the formula VII, and deprotecting the compound shown in the formula VII to obtain the compound alogliptin or the salt thereof.

EP 2 682 110 discloses a multilayer tablet comprising alogliptin benzoate and a production method for alogliptin benzoate. The preparation method comprises the steps of producing 2-((6-chloro-3-methyl-2,4-dioxo-3,4-dihydropyrimidin-1(2H)-yl)methyl)benzonitrile, reacting this with 1.1 equivalents of (R)-3-aminopiperidine dihydrochloride in isopropanol and water at 58-68 °C until the completion of the reaction, adding potassium carbonate and cooling prior to removal of the inorganic salt by filtration. The method further comprises the steps of acidifying the mixture with HCl in THF before cooling and drying to obtain a crystalline solid of (R)-2-((6-(3-aminopiperidin-1-yl)-3-methyl-2,4-dioxo-3,4-dihydropyrimidin-1 (2H)-yl)methyl)benzonitrile, the HCl salt of which is then dissolved in water and washed with isopropyl acetate to remove the dimer. The method further comprises the step of heating the mixture with solid potassium carbonate prior to separating and concentrating the organic phase. The organic phase is then treated with alcohol and passed through a dust removal filter prior to the addition of a solution of benzoic acid in hot ethanol. The method further comprises the steps of cooling the solution and stirring to obtain crystals which are then filtered, washed and dried to obtain crystalline alogliptin benzoate.

The major drawback of the prior art process as described in US'689 Patent is that it involve use of hazardous reagents such as sodium hydride and lithium bromide. Also, the other prior art method requires the protection of the amino compound before reacting, which results in increase in the number of steps thereby making the process cumbersome and uneconomical.

Therefore, there remains a need in the art to develop a safe and cost effective process for the preparation of alogliptin by using economical reagents which makes the process industrially viable and hence, advantageous. The present invention therefore seeks to address these issues. Thus, it is an objective of the present invention to develop a simple and cost effective process employing industrially safe and readily available starting materials.

### SUMMARY OF THE INVENTION:

A cordingly, the present invention provides a process for preparing alogliptin the compound of Formula I and its pharmaceutically acceptable salts using readily available, cost effective, and industrially safe starting materials.

According to primary object of the present invention, there is provided a simple, cost effective and industrially safe process for the preparation of alogliptin free base the compound of Formula I and its pharmaceutically acceptable salt, which comprises the steps of:
i. reacting N-methylbarbituric acid, the compound of Formula III; 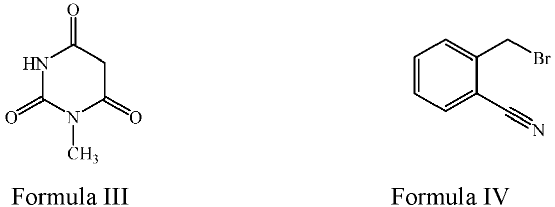 with a halogenating reagent in the presence of a base and a solvent to get an intermediate compound which is reacted in situ with 2-(bromomethyl)benzonitrile, the compound of Formula IV in the presence of a base and a second solvent to obtain 2-[(6-chloro-3-methyl-2,4-dioxo-3,4-dihydropyrimidin-1-(2H)-yl)methyl]benzonitrile, the compound of Formula V; 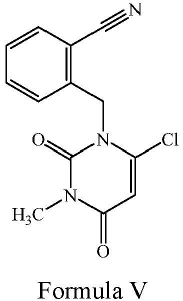
ii. reacting the compound of Formula V obtained in step (a) with (R)-piperidin-3-amine free base or its salt, the compound of Formula VI 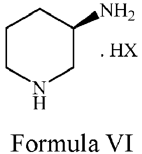 Wherein X is Cl or Br;
   in the presence of an organic solvent to obtain alogliptin free base, the compound of Formula I;

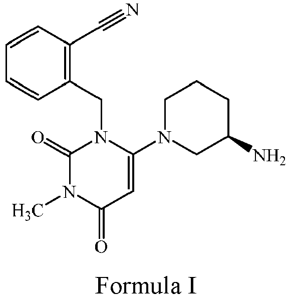

According to another object of the invention, there is provided a process for the preparation of the benzoate salt of alogliptin , the compound of Formula II; comprising the steps of:
i. reacting alogliptin free base, the compound of Formula I prepared by the process as described herein , with an acid to isolate acid addition salt of alogliptin; and
ii. treating the acid addition salt of alogliptin with a base followed by reacting with benzoic acid in the presence of a solvent to isolate alogliptin benzoate salt, the compound of Formula II.

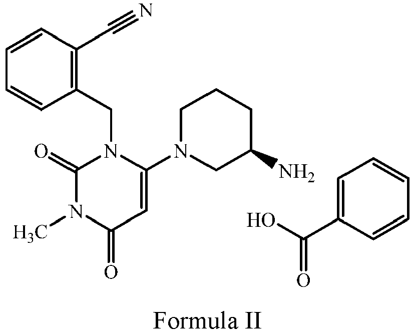

These and the other objects of the present invention will be apparent from the following detailed description.

### DETAILED DESCRITION OF THE INVENTION:

The present invention relates to a process for preparing alogliptin, the compound of Formula I and its benzoate salt, the compound of Formula II.

In an aspect, the present invention relates to a process for the preparation of alogliptin free base, the compound of Formula I, which comprises the steps of:
a) reacting N-methylbarbituric acid, the compound of Formula III; 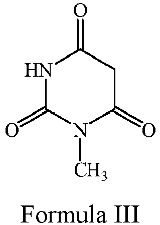 with a halogenating reagent in the presence of a base and a solvent to obtain an intermediate compound which on in situ reaction with 2-(bromomethyl)benzonitrile, the compound of Formula IV; 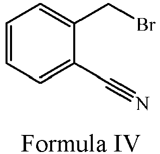 in the presence of a base and a second solvent to obtain 2-[(6-chloro-3-methyl-2,4-dioxo-3,4-dihydropyrimidin-1-(2H)-yl)methyl]benzonitrile, the compound of Formula V; 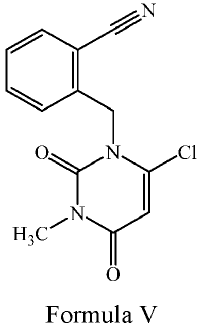
b) reacting the compound of Formula V as obtained in step (a) above with (R)-piperidin-3-amine free base or its salt, the compound of Formula VI 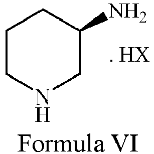 (wherein X is Cl or Br);
   in the presence of an organic solvent to obtain alogliptin free base, the compound of Formula I.

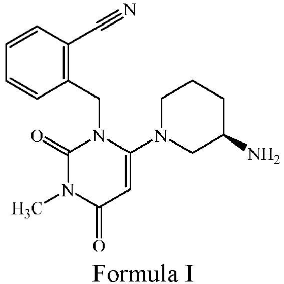

The process as described above further comprises treating alogliptin free base, the compound of Formula I with an acid to isolate acid addition salt of alogliptin; which is treated with a base followed by treating it with benzoic acid to obtain the corresponding benzoate salt of alogliptin, the compound of formula II.

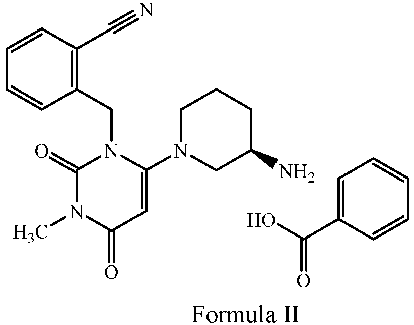

In another aspect, the present invention relates to a process for the preparation of alogliptin benzoate salt, the compound of Formula II, which comprises the steps of:
a) reacting N-methylbarbituric acid, the compound of Formula III with a halogenating reagent in the presence of a base and a solvent to obtain an intermediate compound which is reacted in situ with 2-(bromomethyl)benzonitrile, the compound of Formula IV in the presence of a base and a second solvent to obtain 2-[(6-chloro-3-methyl-2,4-dioxo-3,4-dihydropyrimidin-1-(2H)-yl)methyl]benzonitrile,the compound of Formula V;
b) reacting the compound of Formula V as obtained in step (a) above with (R)-piperidin-3-amine free base or its salt, the compoundof Formula VI in the presence of an organic solvent to obtain alogliptin free base, the compound of Formula I;
c) in situ reacting alogliptin free base, the compound of Formula I as obtained in step (b) with an acid to obtain acid addition salt of alogliptin; and
d) reacting the acid addition salt as obtained in step (b) above with a base followed by reacting with benzoic acid to obtain alogliptin benzoate, the compound of Formula II.

The term "*in situ*" is used herein to mean that within the reaction mixture and without isolation of the intermediate compound.

In an embodiment of the present invention, the halogenating reagent used in step (a) of the process is selected from the group consisting of phosphorous oxychloride, phosphorous trichloride, phosphorous pentachloride, thionyl chloride, oxalyl chloride and phosphorous tribromide.

In an embodiment of the present invention, the halogenating reagent used in step (a) of the process is phosphorous oxychloride.

In an embodiment of the present invention, the reaction of step (a) is carried out in the presence of the solvent selected from the group consisting of toluene, xylene, dichloromethane, dichloroethane, and chlorobenzene or a mixture thereof.

In an embodiment of the present invention, the reaction of step (a) is carried out at a suitable temperature, preferably at a temperature ranging from 30°C to the reflux temperature of the solvent used for the reaction. The most preferred temperature of the reaction is in the range of 85°C to 105°C.

In an embodiment of the present invention, the base used in the reaction step (a) is an organic base selected from the group consisting of N,N-dimethylaniline, pyridine, morpholine, tertiary-butylamine and triethylamine.

In an embodiment of the present invention, after completion of the reaction of step (a), the reaction mixture is cooled to about 10°C, and the resulting reaction mass is diluted with a first solvent selected from the group consisting of methanol, ethanol, isopropyl alcohol, n-butanol, tertiary-butanol and stirred for 1-2 hour. The liquid from the reaction mass is separated and to the residual mass of the intermediate compound, second solvent selected from the group consisting of tetrahydrofuran, N,N-dimethylformamide, dimethylsulfoxide, N-methylpyrrolidine and toluene, is added. The solution of the intermediate compound is further reacted with 2-(bromomethyl)benzonitrile, the compound of Formula IV in the presence of a base at a temperature range of 55°C to 90°C for 4 to 5 hours. The base used for this reaction is selected from the group consisting of N,N-dimethylaniline, pyridine, morpholine, tertiary-butylamine, diisopropylethylamine and triethylamine. The completion of the reaction is monitored by HPLC technique. After completion of the reaction, the solvent is concentrated under reduced pressure to get a residual solid. To the residual solid charged mixture of solvent selected from water methanol, ethanol, isopropanol, n-butanol and tertiary-butanol and is stirred at 10°C to 20°C for one hour. The preferred mixture of solvent selected is water and isopropanol. The solid that is separated out is filtered and washed with isopropanol to obtain pure 2-[(6-chloro-3-methyl-2,4-dioxo-3,4-dihydropyrimidin-1-(2H)-yl)methyl]benzonitrile, the compound of Formula V.

In an embodiment of the present invention, in the reaction of step (b), the compound of Formula V, 2-[(6-chloro-3-methyl-2,4-dioxo-3,4-dihydropyrimidin-1-(2H)-yl)methyl]benzonitrile as obtained in the above step (a) is condensed with (R)-piperidin-3-amine free base or its salt, the compound of Formula VI in the presence of a base, a solvent and optionally in the presence of a suitable catalyst to obtain the desired alogliptin free base, the compound of Formula I.

In an embodiment of the present invention, the base used in the condensation reaction of step (b) is selected from the group consisting of sodium carbonate, potassium carbonate, sodium bicarbonate, potassium bicarbonate, triethylamine and diisopropylethylamine.

In an embodiment of the present invention, the solvent used in the condensation reaction of step (b) is a protic or an aprotic solvent selected from the group consisting of methanol, isopropanol, ethyl acetate, N,N-dimethylformamide, dimethylsulfoxide and N,N-dimethylacetamide; or a mixture thereof. In a preferred embodiment, a mixture of two to three solvents is used in the step (b).

In an embodiment of the present invention, the reaction of step (b) is carried out in the presence of a catalyst selected from potassium iodide, sodium iodide, ammonium iodide and tetrabutylammonium iodide. In a preferred embodiment, the catalyst used for the reaction is potassium iodide.

In an embodiment of the present invention, the reaction of step (b) is carried out at a temperature in the range of 50°C to 90°C. In a preferred embodiment, the reaction is carried out at 65°C to 75°C.

In another embodiment of the present invention, the present invention relates to a process for the preparation of alogliptin benzoate salt, the compound of Formula II,comprising the steps of
i. reacting alogliptin free base the compound of Formula I, obtained according to the process of the present invention with an acid to isolate acid addition salt of alogliptin;
ii. treating the acid addition salt of alogliptin with a base followed by reacting with benzoic acid in the presence of a suitable solvent to isolate alogliptin benzoate salt, the compound of Formula II.

In an embodiment of the present invention, the acid addition salt is selected from the group consisting of hydrochloric acid, sulphuric acid, phosphoric acid and acetic acid. The preferred acid addition salt is hydrochloric acid salt.

In an embodiment of the present invention, the base used in the reaction is selected from the group consisting of sodium carbonate, potassium carbonate, sodium bicarbonate, potassium bicarbonate, triethylamine and diisopropylethylamine. The preferred base used for the reaction is sodium carbonate.

In an embodiment of the present invention, the solvent used in the reaction involving treatment with benzoic acid is selected from the group consisting of methanol, ethanol, 1-propanol, isopropyl alcohol, n-butanol, 2-butanol, tertiary-butanol, ethyl acetate, acetone, methyl ethyl ketone, methyl isobutyl ketone, dichloromethane and cyclohexane; or a mixture thereof.

The isolated alogliptin benzoate salt can be further purified by any conventional purification method known to a person skilled in the art to obtain pure alogliptin benzoate, the compound of Formula II.

The starting material, N-methylbarbituric acid, the compound of Formula III used in the process of the present invention is a readily available commercial compound, and is also relatively cheap in terms of its cost. Thus, use of N-methylbarbituric acid as the starting material renders the process of the present invention cost-effective.

Also, the process of the present invention is advantageous because it avoids use of metal hydride such as sodium hydride as a base thereby rendering the process industrially safe.

The following examples, which fully illustrate the practice of the preferred embodiments of the present invention, are intended to be for illustrative purpose.

### Examples:

### Example 1: Preparation of 2-[(6-chloro-3-methyl-2,4-dioxo-3,4-dihydropyrimidin-1-(2H)-yl)methyl]benzonitrile (the compound of Formula V):

In a dry flask charged toluene (150 ml), N-methylbarbituric acid (50 gm), phosphorous oxychloride (64.5 gm) and N,N-dimethylaniline (20 ml). Heated the reaction mass to 90°C - 95°C and maintained the reaction at this temperature for 2 hours. The reaction progress was monitored by TLC. After completion of the reaction, cooled the reaction mass to 10°C - 15°C and charged methanol (150 ml) in the reaction mass and stirred at this temperature for 1 hour. The reaction mass was allowed to settle and the solvent was removed from the reaction mass. Charged again methanol (50 ml), stirred and separated the solvent from the reaction mixture to get the residual solid mass of the intermediate compound.

To the solution of the intermediate compound charged solvent tetrahydrofuran (300 ml), 2-(bromomethyl)benzonitrile (67.3 gm) and diisopropylethylamine (60.5 gm). Raised the temperature of the reaction mass to 60°C - 65°C and maintained at this temperature for 4 - 5 hours. The reaction progress was monitored by TLC. After the completion of the reaction the solvent was concentrated and to the residual mass charged water (300 ml) and stirred the reaction mass at 25°C - 30°C for 1 hour. Filtered the reaction mass and washed the solid mass with water (50 ml). The filtered wet solid mass was taken in solvent isopropyl alcohol (150 ml) and stirred at 25°C - 30°C for 1 hour. Filtered the solid and washed with isopropyl alcohol (50 ml) and dried the compound to get 2-[(6-chloro-3-methyl-2,4-dioxo-3,4-dihydropyrimidin-1-(2H)-yl)methyl]benzonitrile. Dry weight = 52 gm.

### Example 2: Preparation of 2-[(6-chloro-3-methyl-2,4-dioxo-3,4-dihydropyrimidin-1-(2H)-yl)methyl]benzonitrile (the compound of Formula V):

In a dry flask charged toluene (250 ml), N-methylbarbituric acid (50 gm), phosphorous oxychloride (86.4 gm) and N,N-dimethylaniline (21.3 gm). Heated the reaction mass to 85°C - 90°C and maintained the reaction at this temperature for 5 hours. The reaction progress was monitored by HPLC. After completion of the reaction, cooled the reaction mass to 0°C - 5°C and charged methanol (200 ml) in the reaction mass and stirred at this temperature for 1 hour. The reaction mass was allowed to settle and siphon out solvent from the reaction mass. Charged again methanol (50 ml) stirred and separated the solvent from the reaction mixture to get the residual mass of the intermediate compound.

To the solution of the intermediate compound, charged solvent tetrahydrofuran (200 ml), 2-(bromomethyl)benzonitrile (55.18 gm) and diisopropylethylamine (74.9 ml). Raised the temperature of the reaction mass to 65°C - 70°C and maintained at this temperature for 4 - 5 hours. The reaction progress was monitored by HPLC. After the completion of the reaction the solvent was concentrated and to the residual mass charged mixture of solvents, water and IPA and stirred the reaction mass at 10°C -15°C for 1 hour. Filtered the reaction mass and washed the solid mass with IPA (50 ml), dried the compound to get 2-[(6-chloro-3-methyl-2,4-dioxo-3,4-dihydropyrimidin-1-(2H)-yl)methyl] benzonitrile,the compound of Formula V.
Dry weight = 40 gm.

### Example 3: Preparation of 2-[[6-[(3R)-3-amino-1-piperidinyl]-3,4-dihydro-3-methyl-2,4-dioxo-1(2H)-pyrimidinyl] methyl] benzonitrile hydrochloride (alogliptin hydrochloride):

In a dry flask charged isopropyl alcohol (250 ml), 2-[(6-chloro-3-methyl-2,4-dioxo-3,4-dihydropyrimidin-1-(2H)-yl)methyl]benzonitrile(50 gm), (R)-3-aminopiperidine dihydrochloride (35.16 gm), sodium carbonate (48.06 gm) and potassium iodide (2.50 gm). Heated the reaction mass to 65°C - 70°C and maintained the reaction at this temperature for 12 to 14 hours. The reaction progress was monitored by HPLC. After completion of the reaction, cooled the reaction mass to 45°C - 50°C then filtered it at same temperature followed by isopropyl alcohol (50 ml) wash. Collected the filtrate and concentrated undervacuum at 45°C - 50°C to obtain the residual mass. To this residual mass charged dichloromethane (350 ml) to get the clear solution.

Washed dichloromethane layer using purified water (250 ml x 3) and concentrated solvent maintaining temperature between 38°C to 40°C to remove one volume of solvent quantity charged. Applied cooling and cooled the reaction mass to 25°C to 30°C. Charged slowly the acidic solution of isopropanol hydrochloride maintaining the temperature of the reaction mass between 25°C to 30°C to bring the pH to less than 2. Stirred the reaction mass at 25°C - 30°C for 2 hours. Filtered the reaction mass and washed the wet cake with dichloromethane and dried the product to get 2-[[6-[(3R)-3-amino-1-piperidinyl]-3,4-dihydro-3-methyl-2,4-dioxo-1(2H)-pyrimidinyl]methyl]benzonitrile hydrochloride (alogliptin hydrochloride).
Dry weight = 40 gm.

### Example 4: Preparation of Alogliptin benzoate salt (the compound of Formula II):

In a flask, charged purified water (250 ml), alogliptin hydrochloride (50 gm), sodium carbonate solution (15.5 gm of sodium carbonate in 77.50 ml purified water) and stirred for 30 minutes to get clear solution. Maintain the pH of the reaction solution between 8 to 10, and extract the solution with dichloromethane.

Separated the dichloromethane layer and washed the layer with purified water. After charcoalisation and filtering through hyflow the solvent was concentrated at 35°C to 40°C.

Charged solvent 1-propanol (175 ml) and solution of benzoic acid (16.25 gm) in 1-propanol (100ml) to the reaction mass at 40°C to 45°C to get clear solution which is stirred and maintained the reaction at 55°C to 60°C for 2.0 hours. Cooled the reaction mass to 25°C to 30°C and maintained for one hour. Filtered the reaction mass and washed the solid mass with 1-propanol, dried the compound to obtain pure alogliptin benzoate the compound of Formula II.
Dry weight = 42.50 gm.

## Claims

1. A process for the preparation of alogliptin the compound of Formula I and its pharmaceutically acceptable salt, 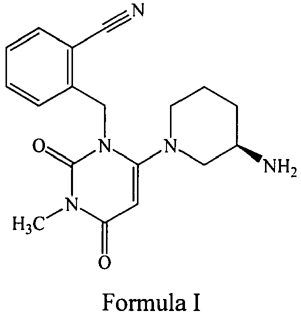 which comprises the steps of:
a) reacting N-methylbarbituric acid, the compound of Formula III; 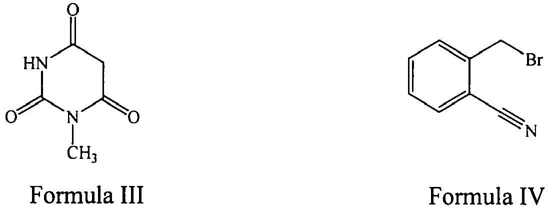 with a halogenating reagent in the presence of a base and a solvent to get an intermediate compound which on in situ condensation reaction with 2-(bromomethyl)benzonitrile, the compound of Formula IV in the presence of a base and a second solvent to obtain 2-[(6-chloro-3-methyl-2,4-dioxo-3,4-dihydropyrimidin-1-(2H)-yl)methyl]benzonitrile, the compound of Formula V; 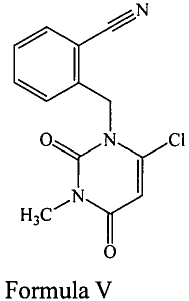
b) reacting the compound of Formula V obtained in step (a) with (R)-piperidin-3-amine free base or its salt, the compound of Formula VI; 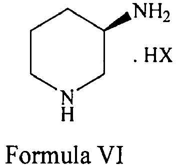 wherein X is Cl or Br;
in the presence of an organic solvent and a base to obtain alogliptin, the compound of Formula I and
c) reacting the compound of formula I obtained in step (b) with an acid in the presence of a solvent to obtain the corresponding acid addition salt of alogliptin.

2. The process according to claim 1, wherein, the halogenating reagent used in step (a) of the process is selected from the group consisting of phosphorous oxychloride, phosphorous trichloride, phosphorous pentachloride, thionyl chloride, oxalyl chloride and phosphorous tribromide
wherein preferably the halogenating reagent is phosphorous oxychloride.

3. The process according to claim 1, wherein, the solvent used in the reaction of N-methylbarbituric acid, the compound of Formula III with halogenating agent in step (a) is selected from the group consisting of toluene, xylene, dichloromethane, dichloroethane, and chlorobenzene or a mixture thereof.

4. The process according to claim 1, wherein, the reaction of step (a) is carried out at a
temperature ranging from 30°C to the reflux temperature of the solvent used wherein, preferably the reaction of step (a) is carried out at temperature ranging from 85°C to 105°C.

5. The process according to claim 1, wherein, the base used in the reaction step (a) is an organic base selected from the group consisting of N,N-dimethylaniline, pyridine, morpholine, tertiary-butylamine, diisopropylethylamine and triethylamine.

6. The process according to claim 1, wherein, the second solvent used in the condensation reaction of step a) is selected from the group consisting of tetrahydrofuran, N,N-dimethylformamide, dimethylsulfoxide, N-methylpyrrolidine and toluene.

7. The process according to claim 1, wherein, the base used in the condensation reaction of step (b) is selected from the group consisting of sodium carbonate, potassium carbonate, sodium bicarbonate, potassium bicarbonate, triethylamine and diisopropylethylamine.

8. The process according to claim 1, wherein, the organic solvent used in the reaction of step (b) is a protic or an aprotic solvent selected from the group consisting of methanol, isopropanol, ethyl acetate, N,N-dimethylformamide, dimethylsulfoxide and N,N-dimethylacetamide; or a mixture thereof.

9. The process according to claim 1, wherein, the reaction of step (b) is carried out in the presence of a catalyst selected from potassium iodide, sodium iodide, ammonium iodide and tetrabutylammonium iodide
wherein preferably the catalyst used is potassium iodide.

10. The process according to claim 9, wherein, the reaction of step (b) is carried out at a temperature in the range of 50°C to 90°C.

11. The process according to claim 1, wherein in step (c), the acid is benzoic acid and the acid addition salt, is alogliptin benzoate.

12. A process for the preparation of alogliptin benzoate salt, the compound of Formula II comprising the steps of:
a) preparing an acid addition salt of alogliptin according to the process of Claim 1; and
b) treating the acid addition salt of alogliptin with a base followed by reacting with benzoic acid in the presence of a solvent to isolate alogliptin benzoate salt, the compound of Formula II.

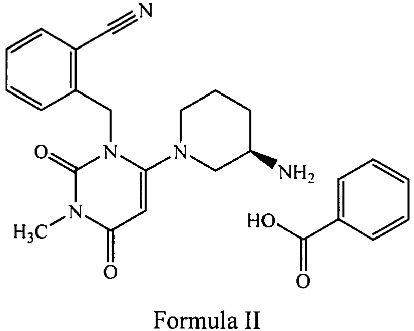

13. The process according to claim 12, wherein, the acid addition salt is selected from the group consisting of hydrochloric acid, sulphuric acid, phosphoric acid and acetic acid.

14. The process according to claim 12, wherein, the base used in the reaction is selected from the group consisting of sodium carbonate, potassium carbonate, sodium bicarbonate, potassium bicarbonate, triethylamine and diisopropylethylamine.

15. The process according to claim 12, wherein, the solvent used for the reaction is selected from the group consisting of methanol, ethanol, 1-propanol, isopropyl alcohol, n-butanol, 2-butanol, tertiary-butanol, ethyl acetate, acetone, methyl ethyl ketone, methyl isobutyl ketone, dichloromethane and cyclohexane; or a mixture thereof.

## Patentansprüche

1. Verfahren für die Herstellung von Alogliptin, der Verbindung von Formel I, und seinem pharmazeutisch verträglichen Salz: 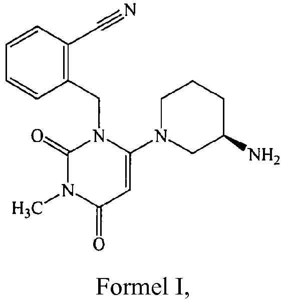 das die folgenden Schritte umfasst:
a) Reagieren von N-Methylbarbitursäure, der Verbindung von Formel III: 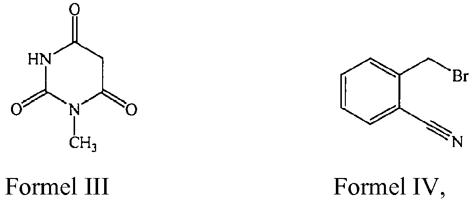 mit einem Halogenierungsreagenz in Gegenwart einer Base und eines Lösungsmittels, um eine Zwischenverbindung zu erhalten, die in situ in einer Kondensationsreaktion mit 2-(Brommethyl)benzonitril, der Verbindung von Formel IV, in Gegenwart einer Base und eines zweiten Lösungsmittels reagiert, um 2-[(6-Chlor-3-methyl-2,4-dioxo-3,4-dihydropyrimidin-1-(2H)-yl)methyl]benzonitril, die Verbindung von Formel V, zu erhalten: 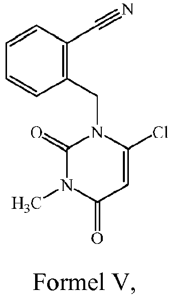
b) Reagieren der Verbindung von Formel V, die im Schritt (a) erhalten wurde, mit der freien Base von (R)-Piperidin-3-amin oder seinem Salz, der Verbindung von Formel VI: 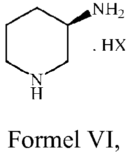 wobei X Cl oder Br ist,
in Gegenwart eines organischen Lösungsmittels und einer Base, um Alogliptin, die Verbindung von Formel I, zu erhalten, und
c) Reagieren der Verbindung von Formel I, die im Schritt (b) erhalten wurde, mit einer Säure in Gegenwart eines Lösungsmittels, um das entsprechende Säureadditionssalz von Alogliptin zu erhalten.

2. Verfahren nach Anspruch 1, wobei das Halogenierungsreagenz, das im Schritt (a) des Verfahrens verwendet wird, aus der Gruppe ausgewählt ist, die aus Phosphoroxychlorid, Phosphortrichlorid, Phosphorpentachlorid, Thionylchlorid, Oxalylchlorid und Phosphortribromid besteht,
wobei bevorzugt das Halogenierungsreagenz Phosphoroxychlorid ist.

3. Verfahren nach Anspruch 1, wobei das Lösungsmittel, das in der Reaktion von N-Methylbarbitursäure, der Verbindung von Formel III, mit dem Halogenierungsmittel im Schritt (a) verwendet wird, aus der Gruppe, die aus Toluol, Xylol, Dichlormethan, Dichlorethan und Chlorbenzol besteht, oder einer Mischung davon ausgewählt ist.

4. Verfahren nach Anspruch 1, wobei die Reaktion von Schritt (a) bei einer Temperatur im Bereich von 30 °C bis zu der Rückflusstemperatur des verwendeten Lösungsmittels durchgeführt wird,
wobei bevorzugt die Reaktion von Schritt (a) bei einer Temperatur im Bereich von 85 °C bis 105 °C durchgeführt wird.

5. Verfahren nach Anspruch 1, wobei die Base, die in dem Reaktionsschritt (a) verwendet wird, eine organische Base ist, die aus der Gruppe ausgewählt ist, die aus N,N-Dimethylanilin, Pyridin, Morpholin, tert-Butylamin, Diisopropylethylamin und Triethylamin besteht.

6. Verfahren nach Anspruch 1, wobei das zweite Lösungsmittel, das in der Kondensationsreaktion von Schritt a) verwendet wird, aus der Gruppe ausgewählt ist, die aus Tetrahydrofuran, N,N-Dimethylformamid, Dimethylsulfoxid, N-Methylpyrrolidin und Toluol besteht.

7. Verfahren nach Anspruch 1, wobei die Base, die in der Kondensationsreaktion von Schritt (b) verwendet wird, aus der Gruppe ausgewählt ist, die aus Natriumcarbonat, Kaliumcarbonat, Natriumbicarbonat, Kaliumbicarbonat, Triethylamin und Diisopropylethylamin besteht.

8. Verfahren nach Anspruch 1, wobei das organische Lösungsmittel, das in der Reaktion von Schritt (b) verwendet wird, ein protisches oder ein aprotische Lösungsmittel ist, das aus der Gruppe, die aus Methanol, Isopropanol, Ethylacetat, N,N-Dimethylformamid, Dimethylsulfoxid und N,N-Dimethylacetamid besteht, oder einer Mischung davon ausgewählt ist.

9. Verfahren nach Anspruch 1, wobei die Reaktion von Schritt (b) in Gegenwart eines Katalysators durchgeführt wird, der aus Kaliumiodid, Natriumiodid, Ammoniumiodid und Tetrabutylammoniumiodid ausgewählt ist,
wobei bevorzugt der verwendete Katalysator Kaliumiodid ist.

10. Verfahren nach Anspruch 9, wobei die Reaktion von Schritt (b) bei einer Temperatur im Bereich von 50 °C bis 90 °C durchgeführt wird.

11. Verfahren nach Anspruch 1, wobei im Schritt (c) die Säure Benzoesäure ist und das Säureadditionssalz Alogliptinbenzoat ist.

12. Verfahren für die Herstellung von Alogliptinbenzoat-Salz, der Verbindung von Formel II, das die folgenden Schritte umfasst:
a) Herstellen eines Säureadditionssalzes von Alogliptin gemäß dem Verfahren nach Anspruch 1; und
b) Behandeln des Säureadditionssalzes von Alogliptin mit einer Base, gefolgt vom Reagieren mit Benzoesäure in Gegenwart eines Lösungsmittels, um Alogliptinbenzoat-Salz, die Verbindung von Formel II, zu isolieren:

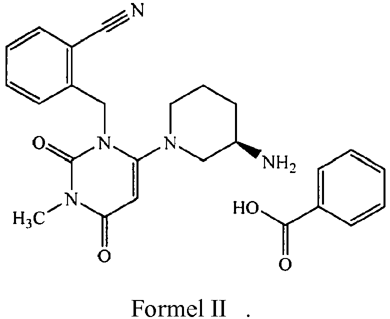

13. Verfahren nach Anspruch 12, wobei das Säureadditionssalz aus der Gruppe ausgewählt ist, die aus Salzsäure, Schwefelsäure, Phosphorsäure und Essigsäure besteht.

14. Verfahren nach Anspruch 12, wobei die Base, die in der Reaktion verwendet wird, aus der Gruppe ausgewählt ist, die aus Natriumcarbonat, Kaliumcarbonat, Natriumbicarbonat, Kaliumbicarbonat, Triethylamin und Diisopropylethylamin besteht.

15. Verfahren nach Anspruch 12, wobei das Lösungsmittel, das für die Reaktion verwendet wird, aus der Gruppe, die aus Methanol, Ethanol, 1-Propanol, Isopropylalkohol, n-Butanol, 2-Butanol, tert-Butanol, Ethylacetat, Aceton, Methylethylketon, Methylisobutylketon, Dichlormethan und Cyclohexan besteht, oder einer Mischung davon ausgewählt ist.

## Revendications

1. Procédé de préparation d'alogliptine, le composé de Formule I, et de son sel pharmaceutiquement acceptable, 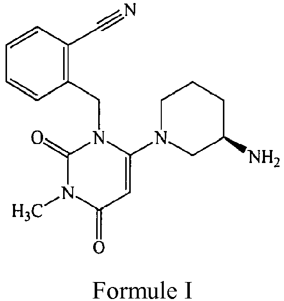 comprenant les étapes consistant à :
a) réagir de l'acide N-méthylbarbiturique, le composé de Formule III 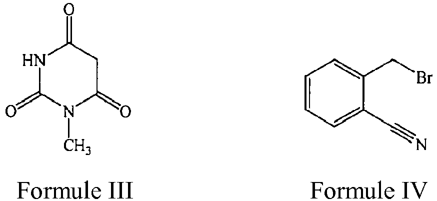 avec un réactif d'halogénation en présence d'une base et d'un solvant afin d'obtenir un composé intermédiaire qui, lors d'une réaction de condensation *in situ* avec du 2-(bromométhyl)benzonitrile, le composé de Formule IV, en présence d'une base et d'un deuxième solvant, permet d'obtenir le 2-[(6-chloro-3-méthyl-2,4-dioxo-3,4-dihydro-pyrimidin-1-(2H)-yl)méthyl]benzonitrile, le composé de Formule V 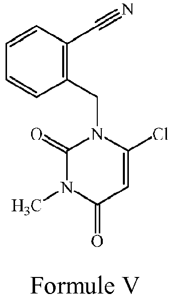
b) réagir le composé de Formule V obtenu dans l'étape (a) avec une base libre de (R)-pipéridine-3-amine ou son sel, le composé de Formule VI 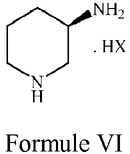 où X est Cl ou Br ;
en présence d'un solvant organique et d'une base afin d'obtenir l'alogliptine, le composé de Formule I, et
c) réagir le composé de Formule I obtenu dans l'étape (b) avec un acide en présence d'un solvant afin d'obtenir le sel d'addition d'acide correspondant de l'alogliptine.

2. Procédé selon la revendication 1, dans lequel le réactif d'halogénation utilisé dans l'étape (a) du procédé est choisi dans le groupe constitué par l'oxychlorure phosphoreux, le trichlorure phosphoreux, le pentachlorure phosphoreux, le chlorure de thionyle, le chlorure d'oxalyle et le tribromure phosphoreux, où préférablement le réactif d'halogénation est l'oxychlorure phosphoreux.

3. Procédé selon la revendication 1, dans lequel le solvant utilisé dans la réaction de l'acide N-méthylbarbiturique, le composé de Formule III, avec un agent d'halogénation dans l'étape (a), est choisi dans le groupe constitué par le toluène, le xylène, le dichlorométhane, le dichloroéthane, et le chlorobenzène, ou un mélange de ceux-ci.

4. Procédé selon la revendication 1, dans lequel la réaction de l'étape (a) est effectuée à une température allant de 30°C à la température de reflux du solvant utilisé, où préférablement la réaction de l'étape (a) est effectuée à une température allant de 85°C à 105°C.

5. Procédé selon la revendication 1, dans lequel la base utilisée dans l'étape de réaction (a) est une base organique choisie dans le groupe constitué par la N,N-diméthylaniline, la pyridine, la morpholine, la tertio-butylamine, la diisopropyléthylamine et la triéthylamine.

6. Procédé selon la revendication 1, dans lequel le deuxième solvant utilisé dans la réaction de condensation de l'étape (a) est choisi dans le groupe constitué par le tétrahydrofurane, le N,N-diméthylformamide, le diméthylsulfoxyde, la N-méthylpyrrolidine et le toluène.

7. Procédé selon la revendication 1, dans lequel la base utilisée dans la réaction de condensation de l'étape (b) est choisie dans le groupe constitué par le carbonate de sodium, le carbonate de potassium, le bicarbonate de sodium, le bicarbonate de potassium, la triéthylamine et la diisopropyléthylamine.

8. Procédé selon la revendication 1, dans lequel le solvant organique utilisé dans la réaction de l'étape (b) est un solvant protique ou aprotique choisi dans le groupe constitué par le méthanol, l'isopropanol, l'acétate d'éthyle, le N,N-diméthylformamide, le diméthylsulfoxyde et le N,N-diméthylacétamide ; ou un mélange de ceux-ci.

9. Procédé selon la revendication 1, dans lequel la réaction de l'étape (b) est effectuée en présence d'un catalyseur choisi parmi l'iodure de potassium, l'iodure de sodium, l'iodure d'ammonium et l'iodure de tétrabutylammonium, où préférablement le catalyseur utilisé est l'iodure de potassium.

10. Procédé selon la revendication 9, dans lequel la réaction de l'étape (b) est effectuée à une température dans la plage allant de 50°C à 90°C.

11. Procédé selon la revendication 1, dans lequel, dans l'étape (c), l'acide est l'acide benzoïque et le sel d'addition d'acide est le benzoate d'alogliptine.

12. Procédé de préparation du sel de benzoate d'alogliptine, le composé de Formule II, comprenant les étapes consistant à :
a) préparer un sel d'addition d'acide d'alogliptine selon le procédé selon la revendication 1 ; et
b) traiter le sel d'addition d'acide d'alogliptine par une base, suivi d'une réaction avec de l'acide benzoïque en présence d'un solvant afin d'isoler le sel de benzoate d'alogliptine, le composé de Formule II,

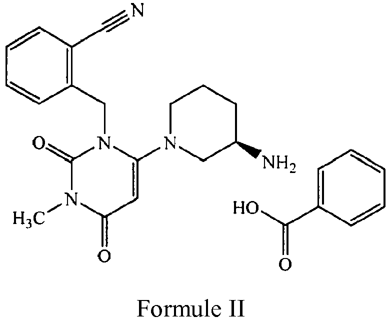

13. Procédé selon la revendication 12, dans lequel le sel d'addition d'acide est choisi dans le groupe constitué par l'acide chlorhydrique, l'acide sulfurique, l'acide phosphorique et l'acide acétique.

14. Procédé selon la revendication 12, dans lequel la base utilisée dans la réaction est choisie dans le groupe constitué par le carbonate de sodium, le carbonate de potassium, le bicarbonate de sodium, le bicarbonate de potassium, la triéthylamine et la diisopropyléthylamine.

15. Procédé selon la revendication 12, dans lequel le solvant utilisé dans la réaction est choisi dans le groupe constitué par le méthanol, l'éthanol, le 1-propanol, l'alcool isopropylique, le n-butanol, le 2-butanol, le tertio-butanol, l'acétate d'éthyle, l'acétone, la méthyléthylcétone, la méthylisobutylcétone, le dichlorométhane et le cyclohexane ; ou un mélange de ceux-ci.
